# EUROPEAN PATENT SPECIFICATION

(11) **EP 1 839 704 B1**
(45) Date of publication and mention of the grant of the patent: **18.05.2011**
(21) Application number: 07104003.4
(22) Date of filing: 13.03.2007
(51) Int. Cl.: A61N 5/06, A61N 5/08

(54) **Device for performing aesthetic treatments**
Vorrichtung zur Durchführung ästhetischer Behandlungen
Dispositif pour effectuer des traitements esthétiques

(30) Priority: 30.03.2006 IT BO20060217
(43) Date of publication of application: 03.10.2007
(73) Proprietor: ESPANSIONE MARKETING S.P.A., 40050 Argelato, Frazione Funo BO (IT)
(72) Inventor: Pomar, Rodolfo, 40067 Pianoro, Frazione Rastignano (IT)
(74) Representative: Modiano, Micaela Nadia

(56) References cited:
- WO-A-2004/098709
- DE-A1- 4 323 936
- DE-U1- 20 013 335
- US-A- 4 909 254
- US-A1- 2004 075 065
- US-A1- 2004 232 359

## Description

The present invention relates to a method for performing aesthetic treatments.

Devices which comprise a plurality of sources capable of emitting light radiation also in the ultraviolet range are universally known as tanning lamps.

Anyone who undergoes a treatment by means of these devices exhibits tanning in the region of the skin that is exposed to the light beam.

Depending on the type of source installed on the device, the intensity of the light beam varies and therefore the effects on the skin of the user also vary.

In practice, there are devices which are capable of inducing a very intense tan in short times (high intensity, high pressure) and others which instead take a long time to obtain noticeable results (low intensity, low pressure).

In order to improve the comfort of users of these devices, air recirculation circuits are provided in order to keep the temperature below unpleasant values, avoiding discomfort conditions for their users.

US 2004/232359 A1 discloses a a skin tanning chamber, comprising at least one light emitting diode emitting a UVA light and, additionally, multiple LEDs of varying types with various characteristic wavelengths are controlled independently to produce an arbitrary light pattern in an arbitrary sequence over time. The chamber can be rigid or flexible. It can be a bed, booth or incorporated into a flexible form, such as a garment or cloth. In one embodiment, the chamber further comprises at least one LED emitting a UVC light, whereby the UVC light sanitizes the chamber surface. Preferably the LED emitting the UVA light is under independent control from the LED emitting UVC light. Furthermore, D1 discloses an additional means to control the peak wavelength is varying the LED ambient cooling mechanism (e.g. fans, thermoelectric cooler, Peltier effect cooling device, and compressor based air conditioners). A UVA LED assembly control system, comprising an analog or digital computer, a suitable algorithm, wavelength sensors, light intensity sensors, skin proximity sensors, and user interface for programming desired results, can be used to calculate the required variations to the current controllers and the duty cycle controllers and the ambient temperature controllers in order to produce a specific peak wavelength from a given set of UVA LEDs.

WO 2004/098709 discloses that a customer is pretreated with NIR light before the actual tanning session.

The aim of the present invention is to provide a method for performing aesthetic treatments which allows to perform tanning treatments as well as aesthetic and curative treatments for the skin simultaneously or separately.

Another object of the present invention is to provide a method for performing aesthetic treatments which also allows to perform treatments such as sauna and Turkish bath.

Another object of the present invention is to provide a method for performing aesthetic treatments which has a low cost and is relatively simple to provide in practice and safe in application.

This aim and these and other objects which will become better apparent hereinafter are achieved by the present device for performing aesthetic treatments, of the type which comprises at least one light source which emits ultraviolet radiation in the direction of a user, characterized in that it comprises at least one second light source which lies proximate to the skin of the user during treatment and works on different frequencies.

Further characteristics and advantages of the invention will become better apparent from the following detailed description of a preferred but not exclusive embodiment of a device for performing aesthetic treatments, according to the claimed method, illustrated by way of non-limiting example in the accompanying drawings, wherein:
Figure 1 is a perspective view of a device for performing aesthetic treatments according to the invention;

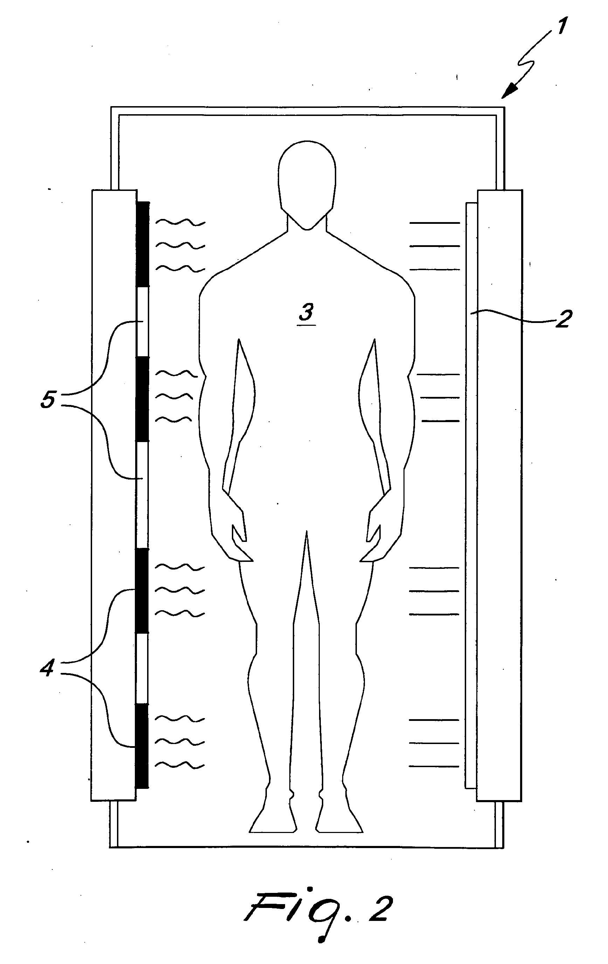

Figure 2 is a schematic view of a device for performing aesthetic treatments according to the invention;
Figure 3 is a front view of a device for performing aesthetic treatments according to the invention which is adapted for treating the face.

With reference to the figures, the reference numeral 1 generally designates a device for performing aesthetic treatments.

The device 1 comprises at least one light source 2, which is capable of emitting ultraviolet radiation in the direction of the user 3: in particular, such source is constituted by tanning lamps of a known type.

There are various types of tanning lamp, which differ in terms of the different power of the emitted light beam: currently, the main distinction is made between high-pressure lamps (which allow intense tanning in short times but are mainly adapted for skins which are already tanned or are scarcely prone to bums) and low-pressure lamps (with which the time required to achieve a visible tan is rather long and which can treat even very fair skin, which bums easily).

The device 1 further comprises at least one second light source, which lies proximate to the skin of the user 3 during the treatment and works on different frequencies.

According to a possible embodiment of particular interest in application, the second light source comprises at least one light-emitting diode 4.

According to another embodiment, which is not an alternative to the preceding one but can be combined with it, the second light source can comprise at least one infrared ray emitter 5: the presence of the emitter 5 ensures intense heating of the exposed skin surface, and in this manner it is possible to obtain treatments of the sauna and Turkish bath type during a tanning cycle.

It can be understood easily that the combination of light sources of different kinds allows to optimize spaces, since a single machine is in fact capable of performing different treatments on the user 3 (both at separate times and simultaneously). The user 3 is also given the possibility to use new intensive treatment cycles which combine various technologies. At the same time, therefore, it is possible to get a tan, have a sauna and eliminate some aesthetic imperfections (or at least make them less evident).

If the light-emitting diodes 4 are adapted to emit a beam of red light, it is in fact possible to use them to stimulate the production of collagen: in this manner, the skin will have a more youthful appearance and will be more elastic. One should not neglect the fact that such a treatment before a tanning treatment prepares the skin, facilitating tanning and avoiding (at least partly) the risk of reddening and bums.

If the diodes 4 are adapted to emit a blue light beam, their effect is to contrast bacterial acne.

If the diodes 4 are instead adapted to emit a yellow beam of light, stimulation of the lymphatic system and of the nervous system occurs. In this case also, an exposure can prepare for a subsequent tanning treatment.

Of course, all the types of light-emitting diode 4 can be arranged along the surface of the device 1 even simultaneously: a computer which manages the device 1 will deal with combining their activation (or excluding one or the other) to perform the chosen treatment.

According to a possible embodiment, the device 1 can be substantially shaped like a tanning bed 6 of the traditional type, on the internal surfaces of which the light sources 4, 5 designed for the various skin treatments are arranged.

In this case, as already mentioned, the operation of the sources 2, 4 and 5 can be simultaneous or alternated, depending on the treatment (or on the treatment cycles) set by the personnel assigned to the device 1.

Of course, and as an alternative to the solution described above, the device 1 can also be shaped substantially like a traditional tanning booth: the same remarks made for the bed 6 apply.

It should be noted that in the case of a tanning booth it might be interesting to provide a supporting platform for the user 3 of the rotating type, so that during rotation every portion of the skin of the user 3 can face for a suitable time each of the active sources 2, 4 and 5.

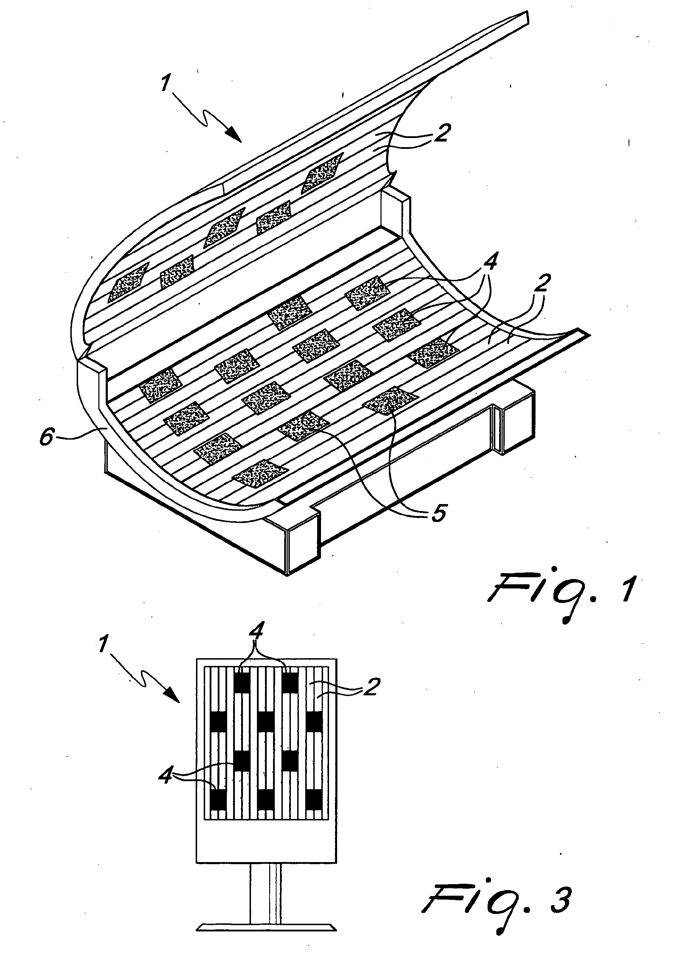

Of course, it is also possible to provide a device 1 which is exclusively suitable for tanning the face, as shown in Figure 3, in which it is possible to combine traditional-type sources 2 (for emitting UVA rays) and sources 4 (in particular light-emitting diodes which are suitable to emit red light beams).

During the execution of aesthetic treatments by using the device 1 according to the invention, it is necessary to place the user 3 proximate to the light sources 2, 4 and 5 with the skin exposed to the sources 2, 4 and 5.

At this point, by using a preferred but not exclusive operating method, it is necessary to activate the light-emitting diodes 4 which are adapted for the emission of a beam of red light in order to stimulate the production of collagen and elastin. In this manner, during this exposure the skin of the user 3 is prepared for tanning. This exposure must be continued for a predefined time which depends on the characteristics of the skin of the user and on any existing tan.

Substantially near the instant when the light-emitting diodes 4 are deactivated (in practice, toward the end of the exposure to the light beam emitted by the diodes 4 or shortly after the diodes 4 have been deactivated), it is necessary to activate the light sources 2, which emit ultraviolet radiation, in the direction of the user 3. At this point, the skin of the user 3 will be particularly receptive (to ultraviolet radiation and to their tanning effect) as a consequence of the previous exposure to the light of the red light-emitting diodes 4.

The exposure of the skin of the user 3 to the light sources 2 occurs for substantially shorter times than in traditional tanning devices, since the production of elastin and collagen is stimulated by the light radiation emitted by the red light-emitting diodes 4, and therefore obtainment of the tan is quicker and more intense and therefore can be obtained in a shorter time, reducing the risk of excessive exposure of the skin to ultraviolet radiation.

It has thus been shown that the invention achieves the proposed aim and objects.

The invention thus conceived is susceptible of numerous modifications and variations.

All the details may further be replaced with other technically equivalent elements.

In the exemplary embodiments shown, individual characteristics, given in relation to specific examples, may actually be interchanged with other different characteristics that exist in other exemplary embodiments.

In practice, the materials used, as well as the shapes and dimensions, may be any according to requirements.

Where technical features are followed by reference signs, those reference signs have been included for the sole purpose of increasing the intelligibility of the claim and accordingly, such reference signs do not have any limiting effect on the interpretation of each element identified by way of example by such reference signs.

## Claims

1. A method for performing aesthetic treatments by using a device comprising at least one light source (2) which emits ultraviolet radiation in the direction of a user (3) and at least one second light source (4, 5) comprising at least one light-emitting diode (4), wherein said second light source (4, 5) is configured to lie proximate to the skin of the user (3) during treatment and works on different frequencies, said light source which emits ultraviolet radiation (2) and said second light source (4, 5) being configured to be active at times different from one another, the method comprising the steps of:
- arranging the user (3) proximate to the light sources (2, 4, 5) with the skin exposed to said sources (2, 4, 5),
- activating the light-emitting diodes (4) which are adapted to emit a red light beam to stimulate the production of collagen and elastin in order to prepare the skin of the user (3) for tanning, for a preset time,
- substantially near the instant when the light-emitting diodes (4) are deactivated, activating the light sources (2) which emit ultraviolet radiation in the direction of the user (3), the user's skin being particularly receptive following the previous exposure to the light of the red light-emitting diodes (4).

## Patentansprüche

1. Ein Verfahren zur Durchführung ästhetischer Behandlungen mit Hilfe einer Vorrichtung, die mindestens eine Lichtquelle (2) umfasst, welche ultraviolette Strahlung in Richtung eines Benutzers (3) aussendet, und mindestens eine zweite Lichtquelle (4, 5), die mindestens eine Leuchtdiode (4) umfasst, wobei die zweite Lichtquelle (4, 5) ausgebildet ist, um während der Behandlung nahe der Haut des Benutzers (3) zu liegen, und mit verschiedenen Frequenzen arbeitet, wobei die Lichtquelle (2), die ultraviolette Strahlung aussendet, und die zweite Lichtquelle (4, 5) ausgebildet sind, um zu Zeiten aktiv zu sein, die sich voneinander unterscheiden, wobei das Verfahren folgende Schritte umfasst:
- Positionierung des Benutzers (3) nahe den Lichtquellen (2, 4, 5), wobei die Haut den Quellen (2, 4, 5) ausgesetzt ist,
- Aktivierung der Leuchtdioden (4), die ausgebildet sind, um einen roten Lichtstrahl auszusenden, um die Produktion von Collagen und Elastin zu stimulieren, um die Haut des Benutzers (3) für eine voreingestellte Zeit auf das Bräunen vorzubereiten,
- im Wesentlichen nahe dem Zeitpunkt, zu dem die Leuchtdioden (4) deaktiviert werden, Aktivierung der Lichtquellen (2), die ultraviolette Strahlung in Richtung des Benutzers (3) aussenden, wobei die Haut des Benutzers nach vorherigem Aussetzen gegenüber dem Licht der roten Leuchtdioden (4) besonders empfänglich ist.

## Revendications

1. Procédé destiné à réaliser des traitements esthétiques au moyen d'un dispositif comprenant au moins une source de lumière (2) qui émet un rayonnement ultraviolet dans la direction d'un utilisateur (3) et au moins une deuxième source de lumière (4, 5) comprenant au moins une diode électroluminescente (4), dans lequel ladite deuxième source de lumière (4, 5) est configurée de façon à se trouver à proximité de la peau de l'utilisateur (3) durant le traitement et fonctionne sur différentes fréquences, ladite source de lumière qui émet un rayonnement ultraviolet (2) et ladite deuxième source de lumière (4, 5) étant configurées de façon à être actives à des périodes de temps différentes l'une par rapport à l'autre, le procédé comprenant les étapes consistant :
- à installer l'utilisateur (3) à proximité des sources de lumière (2, 4, 5) avec la peau exposée aux dites sources (2, 4, 5) ;
- à activer les diodes électroluminescentes (4) qui sont adaptées pour émettre un faisceau de lumière rouge pour stimuler la production de collagène et d'élastine dans le but de préparer la peau de l'utilisateur (3) au bronzage, pendant une période de temps prédéterminée ;
- sensiblement au moment même où les diodes électroluminescentes (4) sont désactivées, le procédé comprenant l'étape consistant à activer les sources de lumière (2) qui émettent un rayonnement ultraviolet dans la direction de l'utilisateur (3), la peau de l'utilisateur étant particulièrement réceptive suite à la précédente exposition à la lumière des diodes émettant une lumière rouge (4).
